# EUROPEAN PATENT SPECIFICATION

(11) **EP 2 565 630 B1**
(45) Date of publication and mention of the grant of the patent: **19.08.2015**
(21) Application number: 11179927.6
(22) Date of filing: 02.09.2011
(51) Int. Cl.: G01N 21/77, G01N 21/78, A61B 5/00

(54) **Dye-doped gelatin-coated optical fibers for in situ monitoring of protease activity in wounds**
Farbstoffdotierte gelatinbeschichtete Glasfasern zur Vor-Ort-Überwachung der Proteaseaktivität in Wunden
Fibres optiques revêtues de gélatine dopée aux colorants pour la surveillance in situ d'activité de protéase dans les plaies

(43) Date of publication of application: 06.03.2013
(73) Proprietor: CSEM Centre Suisse d'Electronique et de Microtechnique SA, 2002 Neuchâtel (CH)
(72) Inventor: Schyrr, Bastien, 1814 La Tour-de-Peilz (CH); Pasche, Stéphanie, 2000 Neuchâtel (CH); Voirin, Guy, 1796 Courgevaux (CH)
(74) Representative: P&TS SA (AG, Ltd.)

(56) References cited:
- EP-A1- 0 864 864
- US-A- 5 828 798
- US-A1- 2004 043 422
- US-A1- 2007 142 762
- US-A1- 2009 156 942
- US-A1- 2009 185 169
- US-A1- 2011 015 591

## Description

### Field

The present invention relates to an optical sensor component, and in particular, but not exclusively, to an optical sensor component which is suitable for monitoring protease activity in wound fluid.

### Description of related art

Wound care is a worldwide growing market. Most research on wound care is focusing on the development of bio-interactive wound dressings on the one side, and on novel strategies for wound therapies on the other side. Whereas those novel developments create a need for better understanding of the wound environment, only few contributions have been looking at in situ monitoring of wounds. Current methods to assess the wound status either consist in clinical observation of the wounds (color, size, depth, odor, etc.), or in the analysis of biopsy sections and swabbed wound exudates samples. Also, current methods for wound care and management are constantly being updated, and more and more relevant markers are listed, resulting from a need for a more quantitative assessment of wounds. In particular, the role of proteases in wounds has been shown to be useful to assess healing status and pathologies in wounds (Reference 1: World Union of Wound Healing Societies (WUWHS), "Principles of best practice: Diagnostics and wounds: A consensus document", Medical Education Partnership (MEP) Ltd, London, 2008).

Commercial devices to monitor wound healing are developed by Aranz Medical (New Zealand), who proposes a tool to assist clinical observations of the wound, and by Ohmedics (UK), who proposes an electrical device that measures wound moisture in real time (WoundSense). In parallel, Sensium (UK) has developed a digital plaster that integrates electrical components. Few labs are considering colorimetric detection of bacteria in wounds, in situ (Rochester (USA) and Fraunhofer (Germany)).

Standard assay for protease activity include those based on substrate zymography, radioisotopes, or fluorogenic substrates (Reference 2: "The role of proteases in wound diagnostics: An expert working group review", London, Wound International, 2011). Zymography is one of the most current methods used to detect protease activity. It relies on the digestion of a gelatin, casein or fibrin substrate, co-polymerized in SDS polyacrylamide gels. In situ zymography allows localized detection of protease activity, directly on the tissue section or cell preparation (Frederiks 2004). Commercially available protease detection kits rely on the specific cleavage of fluorophore-peptide-quencher complexes. The protease activity directly correlates with the fluorescence intensity in solution. In addition, enzyme-linked immunosorbent assays (ELISA), western blot and immuno-histochemistry rely on specific antibodies to quantify proteases. All these techniques are however time-consuming and require specific instrumentation.

Detection of protease activity is extensively studied in the literature, focusing on two strategies. First, a more generic strategy relies on the non-specific digestion of substrates such as gelatin by proteases. An optical biosensing platform was developed for a protease activity assay, based on the SPR property of gold nanoparticles coated with gelatin. Another label-free colorimetric detection method for the detection of gelatinases is based on the degradation of a gelatin layer spin-coated on porous silicon photonic films, which, upon digestion of the gelatin, changes color. The second strategy looks at specific cleavage of a peptide complex, such as detection of protease activity using fluorescence quenching, FRET, or specific cleavage of chromophores on planar optical waveguides. In vivo detection of protease activity has been demonstrated with peptide fluorescent probes.

Most current assays are in vitro methods, and therefore do not allow in vivo monitoring. In contrast, in vivo detection methods are invasive and do not allow monitoring over a long period.

Optical fiber sensors have been widely reported in the literature (Reference 3: I.K. Ilev, I. Gannot, "Fiber optic biosensors", Encyclopedia of Biomaterials and Biomedical Engineering 2005, DOI: 10.1081/E-EBBE-120041886). Optical fibers are interesting candidates for healthcare application, such as for in vivo biosensing. A class of fiber optic sensors relies on the evanescent wave absorption spectroscopy, along the uncladded fiber. In particular, pH sensing optical fibers that measure the absorbence of a colored indicator sensitive to pH, immobilized in a porous film chemically grafted onto the core of a silica optical fiber. The optical fiber chemical sensor is coupled to a (spectro)photometric device for remote, in situ analysis of the acidity.

US2009156942 discloses a method for detection of hospital acquired infections utilizing fluorophore pairs that optically interact with one another according to Forster resonance energy transfer (FRET) or bioluminescence resonance energy transfer (BRET) mechanism. One member of the pair or a cofactor that interacts with an enzyme to form a member of the pair may be tethered to a device by a substrate that is specific for an enzyme expressed by a targeted pathogen.; Upon interaction of the enzyme with the substrate, an optically detectable signal may be altered or initiated, detection of which may then provide information as to the existence of the pathogen at the sit

Document US2004043422 discloses in-situ detection of the presence of a substance or a microbe at a location. The indicator comprises a layer which is susceptible to degradation by the substance or microbe or a first substance associated with the microbe and a signaling layer which is adapted to produce a detectable signal which indicates the presence of the substance or microbe or a second substance associated with the microbe or a further substance which is located at substantially the same location as the substance or microbe.

### Summary of the invention

It is an aim of the present invention to obviate, or mitigate, one or more of the above-mentioned disadvantages.

According to a first aspect of the present invention there is provided, an optical sensor component for use in an optical sensor for monitoring of protease activity in wound fluid, the optical sensor component comprising, a sensing optical fiber comprising an unmodified portion and a modified portion, the modified portion comprising an uncladded portion of the optical fiber which is coated with a coating, the coating having a dye which is configured to absorb light which is transmitted in the uncladded optical fiber, the coating being configured such that it has a predetermined sensitivity to protease such that the coating degrades upon contact with protease, wherein degeneration of the coating decreasing the amount of light which can be absorbed in the sensing optical fiber.

The optical sensor may be suitable for use in situ and continuous of protease activity in wound fluid of a subject e.g. a patient and for continuous monitoring of the subject with large (extended) or deep wounds or with complex wounds, e.g. chronic, surgical, or internal wounds.

The coating may comprise gelatin or natural or synthetic polymers.

Fibers within coating may be configured to have a predefined cross-linking density so that the coating has the predefined sensitivity to degradation rate by protease.

The coating may be configured to have a predefined thickness so that the coating has a predetermined response time sensitivity to protease.

Said dye may comprise chlorophyllin and/or eosin.

According to the further aspect of the present invention there is provided a wound dressing comprising any one of the afore-mentioned optical sensor components.

According to a further aspect of the present invention there is provided an optical sensor device for in situ and continuous monitoring of protease activity in wound fluid, the optical sensor device comprising, a optical sensor component according to any one of the afore-mentioned optical sensor components; a light source in operable cooperation with one end of the sensing optical fiber such that the light generated by the light source can be guided within the sensing optical fiber to provide an optical signal which comprises light which is absorbable by the dye of the sensing optical fiber; a light detector in operable cooperation with one of the ends of the optical fiber so that the light detector can receive the optical signal; a signal processing module configured to process the optical signal received by the light detector to determine the protease activity based on the amount of light in the optical signal received by the light detector.

Glass and polymer optical fibers are modified to allow measuring the activity of proteases in the wound liquid environment (wound fluid). Optical detection relies on the change in absorbance around the core of an optical fiber, connecting the fiber to a light source at one end, and to a light detector at the other end or at the same end. The sensing layer is based on a colored gelatin layer, which, upon digestion by protease enzymes, will degrade. When degradation occurs close to the fiber core, and is thus seen by the evanescent wave of the propagating modes, it will induce a decrease in the fiber absorbance at the wavelength characteristic of the dye in the gelatin. Degradation of the colored gelatin layer is thus converted into an optical signal, which, in turn, is transformed into an electrical signal.

Whereas proof of concept has been demonstrated with gelatin, the layer can be made from other natural or synthetic polymers, for example biocompatible co-polymers comprising specific protease-cleavable parts and non-degradable sections.

The signal processing module may be configured to process the optical signal received by the light detector to determine the protease activity based on strength of the optical signal received by the light detector. The strength of the optical signal measured by the light detector at a given wavelength is related to the change in absorbance of the coating 4 when the coating 4 is being degraded by the protease. In order to minimize the dependency of the measured strength of the optical signal on the power of the light source, a reference optical signal having another wavelength for which the dye 5 is not absorbent can be measured by the light detector.

The optical sensor device may further comprise at least another sensing optical fiber. The optical sensor device may comprise a plurality of sensing optical fiber.

The coating of the sensing optical fiber and the coating of said at least another sensing optical fiber may each be configured have different predetermined sensitivities to protease such that the coating of the sensing optical fiber degrades at a different rate to the rate at which the coating of the at least another sensing optical fiber degenerates, upon contact with the same concentration protease.

The signal processing module may be configured such that it can carry out multiplexing so that the processing module can process, individually, the optical signal received at the light detector from the sensing optical fiber and the optical signal received at the light detector from the at least another sensing optical fiber, to determine the protease activity.

According to a further aspect of the present invention there is provided a method for detecting protease activity in wound fluid, using any one of the afore-mentioned optical sensor devices, comprising: arranging the optical sensor component such that the coating of the sensing optical fiber in contact with wound fluid; transmitting an optical signal from the optical source which is in operable cooperation with one end of the sensing optical fiber, along the sensing optical fiber, to provide the optical signal; detecting the amount of light in the optical signal with the light detector which is in operable cooperation with one of the ends of the optical fiber, during a predetermined measurement time period; using the detected amount of light in the optical signal to determine the protease activity in the wound fluid.

The amount of light in the optical signal detected may comprise the optical strength of the optical signal. The optical strength of the optical signal will indicate the amount of optical light which was absorbed as it transmitted through the sensing optical fiber. The more the coating is degraded, or digested, by the protrease, the less light absorbing dye which is available to absorb light. The amount and rate at which the coating is degenerated will be proportional to the concentration of protease which is in contact with the coating. Accordingly, the optical strength of the optical signal received by the light detector will be proportional to the protease in the wound fluid, and thus proportional to the protease activity.

The optical fiber signal transmitted by the light source may comprise wavelengths at about the maximum absorption peak of the light absorbing dye in the coating.

The optical fiber signal transmitted by the light source may comprise wavelengths corresponding to a minimum or null absorption of the optical signal by the dye in the coating, such as to provide a reference optical signal for said detecting protease activity.

According to a further aspect of the present invention there is provided a method for preparing the optical sensor component, comprising the steps of: removing cladding over the cladded portion of an optical fiber to provide an uncladded portion; coating the uncladded portion with the coating wherein the coating is configured such that it has a predetermined sensitivity to protease such that the coating degrades upon contact with protease; and providing the coating with a light absorbing dye which is configured to absorb light which is transmitted in the uncladded portion, so that degradation of the coating decreases the amount of light which can be absorbed in the uncladded portion. The coating may comprise gelatin. The dye may comprise chlorophyllin and/or eosin.

The method may further comprise, prior to said coating the uncladded portion, activating a surface of the uncladded portion with an amino silane.

### Brief Description of the Drawings

The invention will be better understood with the aid of the description of an embodiment given by way of example only and illustrated by the figures, in which:
Figs. 1 (a) to (c) illustrate a sensing principle which is used in an optical sensor component according to the present invention, for detecting protease activity;
Fig. 2 illustrates steps in preparing an optical sensor component used in an optical sensor device according to the present invention;
Fig. 3 represents a measurement setup which uses an optical sensor component according to an embodiment of the present invention, for monitoring protease activity in the laboratory;
Figs 4 (a) to (d) illustrate the monitoring of MMP-9 with an optical sensor device, showing the decrease in absorbance at 633 nm as a function of time, resulting from digestion of the gelatin layer by various concentrations of MMP-9 protease.

### Detailed Description of possible embodiments

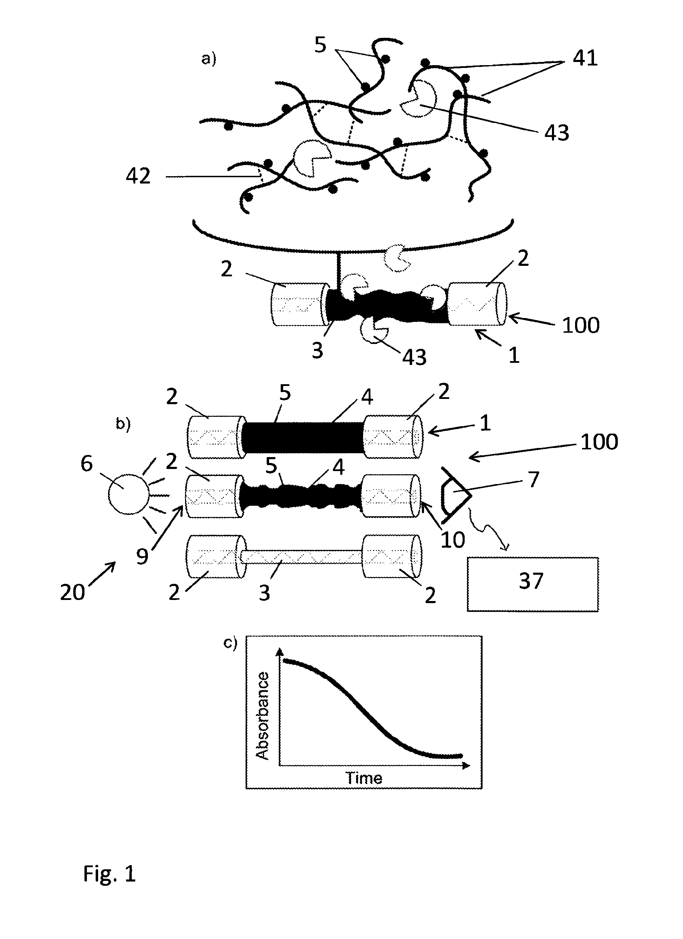

Figs. 1 (a) to (c) illustrate a sensing principle for detecting protease activity with optical component 100 according to an embodiment, which comprises a sensing optical fiber 1. More particularly, Fig. 1 (a) shows an optical component 100 comprising the sensing optical fiber 1 comprising an unmodified portion, here a cladded portion 2, and a modified portion, here an uncladded portion 3. The uncladded portion 3, or the core of the optical fiber 1, is coated with a coating 4 containing a light absorbing, or colored, dye 5. In the exemplary embodiment of Fig. 1 (a), the coating 4 comprises gelatin layer containing chlorophyllin dye used as colored dye 5. The insert of Fig. 1 (a) illustrate en enlarged portion of the coating 4 schematically representing the gelatin 41 with the crosslinks 42, the colored dye 5 and the protease 43. At least the uncladded portion 3 of the sensing optical fiber 1 comprising the coating 4 is destined to be in contact with the wound fluid. The coating 4 has a predetermined sensitivity to protease activity of protease contained in wound fluid. More particularly, the coating 4 comprising the colored dye 5 can be digested by proteases contained in the wound fluid when the uncladded portion 3 of the optical fiber 1 is brought in contact with the wound fluid. Here, the expression "the coating 4 comprising the colored dye 5 can be digested by protease" means that, when in contact with the wound fluid containing protease, the coating 4 comprising the colored dye 5 is degraded or digested at a predetermined degradation rate by the protease.

Fig. 1 (b) illustrates an optical sensing device 20 which uses the sensing optical fiber 1. The digestion of the coating 4 on the sensing optical fiber 1 is also illustrated. The optical sensing device 20 comprises a light source 6 and a light detector 7. Optionally a plurality of light sources 6, light detectors 7 are provided. Each light source 6 may provide light at different wavelength(s). The ratio of absorbance at two wavelengths can be considered, this allows correcting for variations in the absolute light source intensity. Preferably a light source 6 which provides light with wavelengths at about the maximum absorption peak of the light absorbing dye 5 e.g. chlorophyllin is provided and a second light source 6 which provides light at wavelengths for which the dye 5 is substantially not absorbing light. Preferably, a plurality of sensing optical fibers 1 is provided in the optical sensing device 20. Such arrangement will enable protease activity at different parts of the wound fluid to be determined as the plurality of sensing optical fibers 1 can be distributed to different locations in the wound fluid. Optionally the coating 4 in each of the plurality of sensing optical fibers 1 will have a different sensitivity to protease i.e. the coating 4 of some sensing optical fibers 1 will be more susceptible to being digested by protease compared to the coating 4 on other sensing optical fibers 1.

The optical sensing device 20 further comprises a processing module 37 which is in operable communication with the light detector 7. The processing module 37 can process the light received at different wavelengths by the light detector 7 to determine the protease activity within a wound fluid. The processing module 37 does this, for example, by correlating the amount of light received by the light detector 7, with a known corresponding level of protease activity. Here, the level of protease activity corresponding to different amounts of light can be predetermined in a calibration step, for example, which is carried out before the optical sensing device 20 is applied to a wound fluid. For example, the processing module 37 may comprise a table which has predetermined correlations between the light strength and protease activity. The processing module 37 should preferably be adapted to allow easy interfacing/cooperation with the optical fiber sensor 1. The processing module 37 may also be configured to be in operable communication with a plurality of light detectors 7 which cooperate with a plurality of sensing optical fibers 1 or a single light detectors 7 which cooperates with a plurality of sensing optical fibers 1; this will allow the protease activity in different areas of the wound fluid to be determined.

The processing module 37 may be configured to carry out multiplexing so as to enable selecting of one of a plurality of different light detectors 7 and/or sensing optical fibers 1 from which to take a light reading; thus allowing the user to selectively address sensing optical fibers 1 which are in different locations in the wound fluid and/or sensing optical fibers 1 which have different sensitivity to protease activity.

Preferably the processing module 37 will be configured to communicate wirelessly with the one or more light detectors 7 and sensing optical fibers 1. This will improve the wearability of the optical sensing device 20. Additionally, the processing module 37 will preferably comprise a user interface which will display results (e.g. a measure of protease activity) and will allow a user to easily control the optical sensing device 20 (e.g. select from which of a plurality of light detectors 7 and sensing optical fibers 1 from which the processing module 37 is to take a light reading).

In use, a light source 6 is placed in operable cooperation with a first end 9 of the sensing optical fiber 1 such as to form an optical signal which is transmitted along the sensing optical fiber 1. A light detector 7 is placed in operable cooperation with a second end 10 of the sensing optical fiber 1 such as to detect the optical signal. The sensing optical fiber 1 is then positioned so that the coating 4 is in contact with wound fluid. Protease contained in the wound fluid will digest the coating 4. As the coating 4 is digested the amount of light absorbing dye 5 in sensing optical fiber 1 is reduced; accordingly the amount of light which is transmitted through the sensing optical fiber 1 and received at the light detector 7, will be increased. Fig. 1 (c) represent schematically the decrease in absorbance in time as the coating 4 is degraded under the action of protease. The amount of the coating 4 which is digested is proportional to the amount protease, or the concentration of protease, contained in the wound fluid; accordingly, the amount of light which is received by the light detector 7 is proportional to the protease contained in the wound fluid. Thus, the amount of light received at the light detector 7 can be used to determine the protease activity within the wound fluid. Alternatively, the light source 6 and light detector 7 can be placed in operable cooperation with the same end 9, 10 of the sensing optical fiber 1. In the latter configuration, the optical signal should be reflected at the other end 10, 9 of the sensing optical fiber 1.

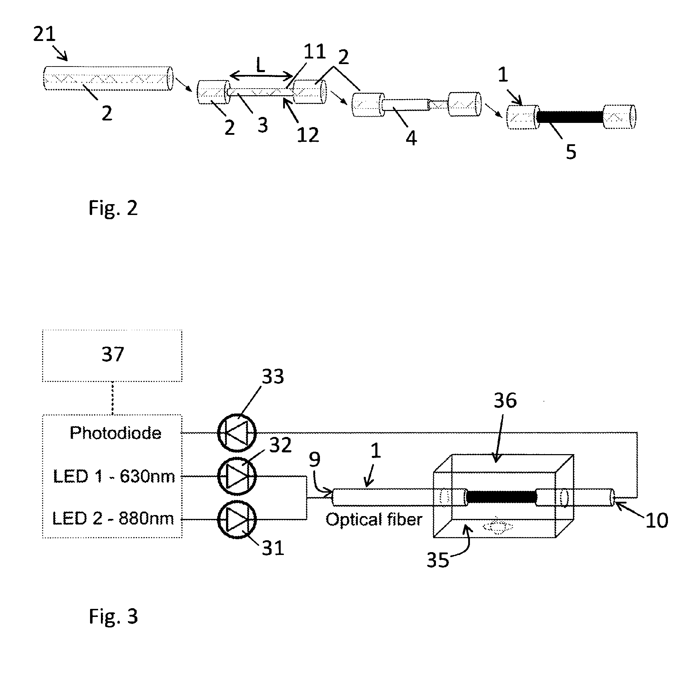

Fig. 2 illustrates preparation of the sensing optical fiber 1 used in the optical sensing device 20 shown in the figures 1(a) to 1(c). To prepare the sensing optical fiber 1, fiber cladding 2 on an optical fiber 21 is removed over a defined length 'L', to define an uncladded portion 3, or modified portion 3, which comprises an exposed optical fiber element 11. In the modified portion 3, the propagating modes of the light which is transmitted through the sensing optical fiber 1 can interact with (is accessible to) the coating 4. Preferably the defined length 'L' is up to 10 cm. Preferably, the optical fiber element 11 comprises glass or polymer with a diameter of 0.1 to 1 mm. Preferably, a surface 12 of the exposed optical fiber element 11 is activated with an amino silane, typically aminopropylmethoxsilane (APTMS) deposited using molecular vapor deposition (MVD) technology.

The uncladded portion 3 is then coated with a coating 4 which comprises gelatin. The gelatin may be sourced from fish or from pork. The gelatin may be mixed with glutaraldehyde to cross-link the gelatin structure, and thus increase its mechanical resistance prior to being coated on the uncladded portion 3. Preferably the coating is achieved by dip-coating the uncladded portion 3 in gelatin. The coating 4 preferably will have a thickness ranging from 0.1 to 2 microns. However it will be understood that the coating 4 may have any suitable thickness. The coating 4 thickness can be tuned by varying the spin-coating velocity. Different coating 4 thicknesses are considered to tune the sensitivity of the detection, targeting different amounts/concentrations of proteases.

The coating 4 is then stained, or doped, with a light absorbing dye 5 which in this particular example is a chlorophyllin dye 5. However, it will be understood that any other suitable dye could be used, for example eosin. Staining or doping of the coating 4 is preferably done using succinimide ester covalent binding. In fact, any other suitable dye 5 can comprise any dye that is light absorbing and possess the following properties: biocompatible; adapted to be incorporated into the coating 4, for example through covalent bonding; ability to not interfere with the activity of protease and with wound healing. After coating, the sensing optical fiber 1 can be sterilized using a conventional sterilization method. For example, the sensing optical fiber 1 can be packaged in ethylene oxide (EO) permeable bag and exposed to EO gas at predetermined sterilization temperature. The sterilization process can be optimized to ensure the activity of the coating 4.

Other methods of preparing the sensing optical fiber 1 are also possible. In an embodiment not represented, the preparation method of the optical fiber 1 comprises heating the optical fiber 1 and pulling on both ends 9, 10 of the optical fiber 1 to obtain a tapered fiber portion (the modified portion 3) having a lower diameter. The cladding and the core in the tapered fiber portion have a proportionally smaller diameter than in the rest of the optical fiber 1 (the unmodified portion 2), making the evanescent wave of the propagating modes of the light which is transmitted through the sensing optical fiber 1 to extend outside the cladding. The tapered fiber portion can then be coated with the coating 4. In an alternative preparation method, a small portion of the cladding and the core can be removed, for example using a drill. The cladding portion and core can be removed during or after fabrication of the fiber 1. The removed portion (the modified portion 3) can then be filled with the coating 4.

Fig. 3 represents a measurement setup for monitoring protease activity in the laboratory, according to an embodiment. More particularly, Fig. 3 schematically shows the use of two light sources in the form of LEDs 31, 32. The LEDs 31, 32 are preferably broadband or several single wavelength LEDs.

The LEDs 31, 32 are in optical communication with one end 9 of the sensing optical fiber 1, and the light detector 7 in the form of a spectrometer or photodiode 33 is in optical communication with the other end 10 of the sensing optical fiber 1. The LEDs 31, 32 generate light which forms the optical signal which is transmitted along the sensing optical fiber 1 and detected by the light detector 7, 33 in the form of a spectrometer or photodiode 33. Alternatively, the LEDs 31, 32 and light detector 7, 33 could also be placed in optical communication with the same end 9, 10 of the sensing optical fiber 1. In this arrangement, the measurement setup can comprise a beam splitter in optical communication with one end 9, 10 of the optical fiber 1 such as to transmit light in the fiber 1 and collect light coming from the fiber 1 and sent it to the light detector 7, 33. The beam splitter can comprise an optical fiber splitter or comprise conventional optical components.

In an embodiment, the light source 6 provides light at a first wavelength at about the maximum absorption peak of the light absorbing dye 5 and the second light source 6 provides light at a second wavelength for which the dye 5 is substantially not absorbing light. Here, collecting the optical signal at the first wavelength of maximum absorbance allows detecting changes in the absorbance around the uncladded part 3 of the fiber 1, and this for measuring the degradation of the coating 4 due to digestion by the protease contained in the wound fluid. Collecting the signal at the second non-absorbing wavelength provides a reference for the detection. Indeed, at the second wavelength, the signal should not vary with the degradation of the coating 4, and can thus be used correcting possible variations in the light source intensity and/or other measurement artifacts.

In the example of Fig. 3, the portion of the sensing optical fiber 1 which comprises the coating 4 is disposed in a recipient, for example a flow cell 36, such as to be immersed in a test liquid 35 containing protease. The coating 4 (which comprises gelatin stained or doped with light absorbing dye 5 which in this case is chlorophyllin dye 5) is degenerated by proteases present in the liquid 35, which progressively will degenerate the entire coating 4, thus resulting in a loss of absorbance around the sensing optical fiber 1. Degradation of the coating 4 will result in a change in the optical signal which is received by the light detector 7, 33. The flow cell 36 is preferably sealed around the optical fiber 1 such as to avoid leakage of the liquid 35. The light source, or LEDs 31, 32, can be in optical communication with one end 9 of the sensing optical fiber 1 via a SMA connector and fiber-to-fiber connectors, or any suitable connectors.

As the coating 4 is degraded the amount of light absorbing dye 5 in sensing optical fiber 1 is reduced; accordingly the amount of light which is transmitted through the sensing optical fiber 1 and received at the spectrometer or photodiode 33, is increased. The amount of the coating 4 which is digested is proportional to the amount protease contained in the test liquid 35; accordingly, the amount of light which is received by the spectrometer or photodiode 33, is proportional to the protease contained in the test liquid 35. Thus, the amount of light received at the spectrometer or photodiode 33 can be used to determine the protease activity within the test liquid 35 or within wound fluid.

The processing module 37 is in operable communication with the spectrometer or photodiode 33. The processing module 37 can process the light received by the spectrometer or photodiode 33 to determine the protease activity within the test liquid 35. The processing module 37 does this, for example, by correlating the amount of light received by the spectrometer or photodiode 33, with a known corresponding level of protease activity; for example the processing module 37 may comprise a table which has predetermined correlations between the amount of light and protease activity. The processing module 37 should preferably be adapted to allow easy interfacing/cooperation with the optical fiber sensor 1.

Trypsin and the matrix metallopeptidase 9 (MMP-9) were selected as a test liquid 35 to demonstrate feasibility of the monitoring fibers. In view of the present invention relevance to wound healing, MMP-9 concentration corresponding to the concentration typically present in chronic wounds: 10-250 µg/mL (normal wounds < 1 µg/mL), was used.

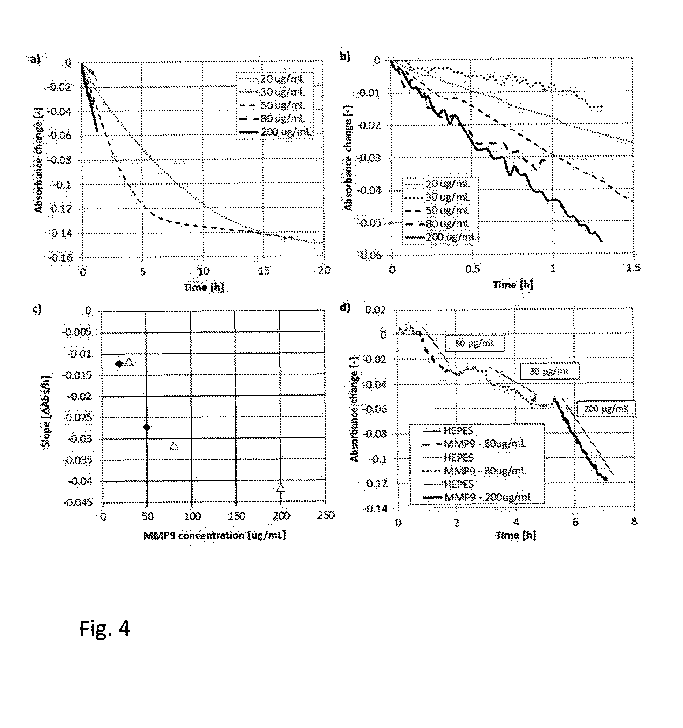

As illustrated in Figs. 4 (a) to (d) the rate of degradation of the coating 4 depends on the concentration of MMP-9 in solution. In order to correct for variations in the absolute light source intensity, the ratio of absorbance at two wavelengths is considered: a first LED at 633 nm corresponds to an absorption peak of chlorophyllin, whereas a second LED at 880 nm does not show any variation in absorbance.

More particularly, Figs 4 (a) to (d) illustrate the monitoring of MMP-9 with the optical fiber sensor 1, showing the decrease in absorbance at 633 nm as a function of time, resulting from digestion of the coating 4 by various concentrations of MMP-9 protease. Fig. 4 (a) provides a graph of absorbance over time (measured in Hours) for different concentrations of MMP-9. Fig. 4 (b) shows the enlargement of the first hour of the graph shown in Fig. 4(a). Fig. 4 (c) provides a graph of the slopes of the lines shown Fig. 4(a) for different MMP-9 protease concentrations. It is clear from the graph of Fig. 4 (c) that there is a variation of the initial slope for digestion as a function of MMP-9 concentration. Fig. 4 (d) provides a graph of absorbance over time for continuous monitoring, for changing MMP-9 concentrations.

The rate of digestion and /or degradation of the coating 4 for a defined protease concentration can be tuned by changing the cross-linking density of fibers e.g. gelatin fibers, in the coating 4. The cross-linking density can be changed by varying the concentration of cross-linker molecules in the coating 4, for example, in a liquid solution that is deposited on the uncladded portion 3. Thus the sensitivity range of the coating 4 can be tuned by adjusting the cross-linking density. Additionally, increasing the thickness of the coating 4 will postpone the digestion and/or degradation of the coating 4, or delay a predetermined response time of the coating 4 to protease, and will extend the lifetime of the optical sensing fiber 1. The thickness of the coating 4 can be increased by varying the conditions for depositing the coating 4 on the uncladded portion 3. For example, a thicker coating 4 can be obtained by dip-coating the uncladded portion 3 at an increased speed, or/and by repeating the dip-coating operation one or several times (multi-layered coating 4). A thicker coating 4, however, will result in a time delay before changes in the absorbance around the uncladded part 3 of the fiber 1 can be detected. Indeed, said changes will be detected for the coating 4 being substantially thinner than a critical thickness corresponding, for example, to a region of propagation of the evanescent wave of the optical signal. The time delay thus corresponds to the time required for degrading the coating 4 until it reaches a thickness corresponding substantially to the critical thickness. The predetermined response time of the coating 4 will then be larger when the thickness of the coating 4 is larger.

Concentrations of MMP-9 between 10 and 200 µg/mL can therefore be measured in real time over a period of 5 to 10 hours. The sensitivity of the sensing optical fibers can be tuned by changing the coating properties (e.g. cross linking of the gelatin or adjusting the thickness of the coating). Additionally, having several optical sensing fibers 1 with different sensitivities in parallel will extensively extend the lifetime of an optical sensing device 20 e.g. may extend lifetime up to 24 hrs.

The influence of the light absorbing dye 5 can be demonstrated by performing similar tests on an optical fiber sensor 1 which does not have its gelatin coating stained or doped with light absorbing dye 5. In the absence of light absorbing dye 5, degradation of the coating 4 will yield no detectable change in the absorbance around the uncladded part 3 and the degradation of the coating 4 cannot be detected.

The coating 4 which comprises gelatin and light absorbing dye 5 has been shown to be stable for at least 3 months in air and in water.

The exemplary measurement setup of Fig. 3 can be used for carrying out in vitro tests in human serum, before testing the optical fiber sensor 1 in vivo. Moreover, the measurement setup of Fig. 3 can be used for the calibration of the optical fiber sensor 1.

Several commercial uses for an optical sensing device 20 and/or optical fiber sensor 1 as described above, for monitoring protease activity, are foreseen in various sectors, in particular, but not exclusively, in the sector of the wound care industry, where such devices/sensors can be used to monitor wound healing. Such devices/sensors can be integrated in wound dressings, or combined with wound therapies, thus making the devices/sensors also useful to the biotechnology/pharmaceutical industry.

A broad range of applications for real-time monitoring of proteases in wounds, using the optical sensing device 20 and/or optical fiber sensor 1 as described above, can be envisioned, such as: Chronic wounds (e.g. ulcers); internal wounds; burns and large acute wounds; skin grafts; and clinical studies.

Various modifications and variations to the described embodiments of the invention will be apparent to those skilled in the art without departing from the scope of the invention as defined in the appended claims. Although the invention has been described in connection with specific preferred embodiments, it should be understood that the invention as claimed should not be unduly limited to such specific embodiment.

### Reference numbers

- 1: sensing optical fiber
- 2: cladded portion
- 3: uncladded portion
- 4: coating
- 41: gelatin
- 42: crosslink
- 43: protease
- 5: dye, chlorophyllin
- 6: light source
- 7: light detector
- 9: first end
- 10: second end
- 11: exposed optical fiber element
- 12: surface of the exposed optical fiber element
- 20: optical sensor device
- 21: optical fiber
- 31: LEDs
- 32: LEDs
- 33: spectrometer or photodiode
- 35: test liquid
- 36: recipient
- 37: signal processing module
- 100: optical component

## Claims

1. An optical component (100) for use in an optical sensor for monitoring of protease activity in wound fluid, the optical component (100) comprising:
a sensing optical fiber (1) comprising an unmodified portion (2) and a modified portion (3), the modified portion (3) comprising a fiber core (3) which is coated with a coating (4), the coating (4) being configured such that it degrades upon contact with protease and being configured to have a predetermined sensitivity to protease which is that the coating (4) degrades at a predetermined rate upon contact with protease,
**characterised by** the coating (4) having a dye (5) which is configured to absorb light which is transmitted in the fiber core (3), wherein degradation of the coating (4) decreases the amount of light which can be absorbed in the sensing optical fiber (1).

2. An optical component (100) according to claim 1, wherein the coating (4) comprises gelatin or natural or synthetic polymers.

3. An optical component (100) according to claim 1 or 2, wherein fibers within coating (4) are configured to have a predefined cross-linking density so that the coating (4) has the predetermined sensitivity to degradation rate by protease.

4. An optical component (100) according to any one of claim 1 to 3, wherein the coating (4) is configured to have a predefined thickness so that the coating (4) has a predetermined response time sensitivity to protease.

5. An optical component (100) according to any preceding claim, wherein said dye (5) comprises chlorophyllin (5) and/or eosin.

6. A wound dressing comprising an optical component (100) according to any one of claims 1 to 5.

7. An optical sensor device (20) for in situ and continuous monitoring of protease activity in wound fluid, the optical sensor device (20) comprising,
an optical component (100) according to any one of claims 1 to 5; a light source (6) in operable cooperation with one end (9, 10) of the sensing optical fiber (1) such that the light generated by the light source (6) can be guided within the sensing optical fiber (1) to provide an optical signal which comprises light which is absorbable by the dye (5) of the sensing optical fiber (1);
a light detector (7) in operable cooperation with one of the ends (9, 10) of the optical fiber (1) so that the light detector (7) can receive the optical signal;
a signal processing module (37) configured to process the optical signal received by the light detector (7) to determine the protease activity based on the amount of light in the optical signal received by the light detector.

8. An optical sensor device (20) according to claim 7, further comprising at least another sensing optical fiber (1).

9. An optical sensor device (20) according to claim 8, wherein the coating (4) of the sensing optical fiber (1) and the coating (4) of said at least another sensing optical fiber (1), are each configured to have different predetermined sensitivities to protease, such that the coating (4) of the sensing optical fiber (1) degrades at a different rate to the rate at which the coating (4) of the at least another sensing optical fiber (1) degenerates, upon contact with the same concentration of protease.

10. An optical sensor device (20) according to any claim 8 or 9 wherein the signal processing module (37) is configured such that it can carry out multiplexing so that the processing module can process, individually, the optical signal received at the light detector (7) from the sensing optical fiber (1) and the optical signal received at the light detector (7) from the at least another sensing optical fiber (1), to determine the protease activity.

11. A method for detecting protease activity in wound fluid, using the optical sensor device (20) according to one of claims from 7 to 10, comprising:
arranging the optical component (100) such that the coating (4) of the sensing optical fiber (1) is in contact with wound fluid;
transmitting an optical signal from the optical source (6) which is in operable cooperation with one end of the sensing optical fiber (1), along the sensing optical fiber (1), to provide the optical signal;
detecting the amount of light in the optical signal with the light detector (7) which is in operable cooperation with one of the ends (9, 10) of the optical fiber (1), during a predetermined measurement time period;
using the detected amount of light in the optical signal to determine the protease activity in the wound fluid.

12. Method according to claims 11, wherein said optical fiber signal transmitted by the light source (6) comprises wavelengths at about the maximum absorption peak of the light absorbing dye (5) in the coating (4).

13. Method according to the claim 11 or 12, wherein the optical signal transmitted by the light source (6) comprises wavelengths corresponding to the minimum absorption of the light absorbing dye (5) in the coating (4), such as to provide a reference optical signal for said detecting protease activity.

14. A method for preparing the optical component (100) according to claim 1, comprising the steps of:
removing cladding over a cladded portion (2) of an optical fiber (21) to provide the fiber core (3);
coating the fiber core (3) with coating (4), wherein the coating is configured such that it degrades upon contact with protease and being configured to have a predetermined sensitivity to protease which is that the coating (4) degrades at a predetermined rate upon contact with protease; and
providing the coating (4) with a light absorbing dye (5) which is configured to absorb light which is transmitted in the fiber core (3), so that degradation of the coating decreases the amount of light which can be absorbed in the fiber core (3).

15. Method according to claim 14, further comprising the step of, prior to said coating the fiber core (3), activating the surface of the fiber core (3) with an amino silane.

## Patentansprüche

1. Eine optische Komponente (100) zur Verwendung in einem optischen Sensor für die Überwachung von Proteaseaktivität in Wundfluiden, die optische Komponente (100) aufweisend:
eine erfassende optische Faser (1) aufweisend einen unveränderten Bereich (2) und einen veränderten Bereich (3), wobei der veränderte Bereich (3) einen Faserkern (3) aufweist, der mit einer Beschichtung (4) beschichtet ist, wobei die Beschichtung (4) so ausgebildet ist, dass sie sich bei Kontakt mit einer Protease zersetzt und ausgebildet ist, eine bestimmte Sensitivität auf Protease hat, die so ist, dass die Beschichtung (4) mit einer vorbestimmten Rate bei Kontakt mit Protease zerfällt,
**dadurch gekennzeichnet, dass** die Beschichtung (4) ein Farbe (5) hat, die ausgebildet ist Licht, das in dem Faserkern (3) übertragen wird, zu absorbieren, wobei der Zerfall der Beschichtung (4) die Lichtmenge, die in der erfassenden optischen Faser (1) absorbiert werden kann, reduziert.

2. Eine optische Komponente (100) nach Anspruch 1, wobei die Beschichtung (4) Gallerte oder natürliche oder synthetische Polymere aufweist.

3. Eine optische Komponente (100) nach Anspruch 1 oder 2, wobei Fasern mit Beschichtung (4) ausgebildet sind, eine vordefinierte Vernetzungsdichte zu haben, so dass die Beschichtung (4) eine vordefinierte Sensitivität bezüglich der Zerfallsrate bei Protease hat.

4. Eine optische Komponente (100) nach einem der Ansprüche 1 bis 3, wobei die Beschichtung (4) ausgebildet ist, eine vordefinierte Dicke zu haben, so dass die Beschichtung (4) eine vordefinierte Antwortzeitsensitivität auf Protease hat.

5. Eine optische Komponente (100) nach einem der vorigen Ansprüche, wobei die Farbe (5) ein Chlorophyllin (5) und/oder Eosin aufweist.

6. Ein Wundverband aufweisend eine optische Komponente (100) nach einem der Ansprüche 1 bis 5.

7. Eine optische Sensorvorrichtung (20) für in situ und kontinuierliche Überwachung von Proteaseaktivität in einem Wundfluid, wobei die optische Sensorvorrichtung (20) aufweist:
eine optische Komponente nach einem der Ansprüche 1 bis 5;
eine Lichtquelle (6) gekoppelt mit einem Ende (9, 10) der erfassenden optischen Faser (1) so dass das von der Lichtquelle (6) erzeugte Licht innerhalb der erfassenden optischen Faser (1) geleitet werden kann, um ein Lichtsignal bereitzustellen, das Licht aufweist, das von der Farbe (59) der erfassenden optischen Faser (1) absorbierbar ist;
ein Lichtdetektor (7) gekoppelt mit einem der Enden (9, 10) der optischen Faser (1), so dass der Lichtdetektor (7) das optische Signal empfangen kann;
ein Signalverarbeitungsmodul (37) ausgebildet das von dem Lichtdetektor (7) empfangene optische Signal zu verarbeiten, um die Proteaseaktivität basierend auf der Lichtmenge in dem von dem Lichtdetektor empfangenen optischen Signal zu bestimmen.

8. Eine optische Sensorvorrichtung (20) nach Anspruch 7, weiter aufweisend mindestens eine weitere erfassende optische Faser (1).

9. Eine optische Sensorvorrichtung (20) nach Anspruch 8, wobei die Beschichtung (4) der erfassenden optischen Faser (1) und die Beschichtung (4) der mindestens einen weiteren erfassenden optischen Faser (1) jeweils ausgebildet sind, verschiedene vorbestimmte Sensitivitäten auf Protease zu haben, so dass die Beschichtung (4) der erfassenden optischen Faser (1) bei Kontakt mit der gleichen Konzentration von Protease sich mit einer unterschiedlichen Rate zersetzt als die Rate, mit der sich die Beschichtung (4) der mindestens einen weiteren erfassenden optischen Faser (1) zersetzt.

10. Eine optische Sensorvorrichtung (20) nach Anspruch 8 oder 9, wobei das Signalverarbeitungsmodul (37) so ausgebildet ist, dass es Multiplexing ausführen kann, so dass das Verarbeitungsmodul das am Lichtdetektor (7) empfangene optische Signal von der erfassenden optischen Faser (1) und das am Lichtdetektor (7) empfangene optische Signal von der mindestens einen weiteren erfassenden optischen Faser (1) individuell verarbeiten kann, um die Proteaseaktivität bestimmen zu können.

11. Ein Verfahren zum Detektieren einer Proteaseaktivität in einem Wundfluid unter Verwendung einer optischen Sensorvorrichtung (20) nach einem der Ansprüche 7 bis 10, aufweisend:
Anordnen der optischen Komponente (100) so, dass die Beschichtung (4) der erfassenden optischen Faser (1) in Kontakt mit dem Wundfluid ist;
Übertragen eines optischen Signals von der optischen Quelle (6), die ausgebildet ist mit einem Ende der erfassenden optischen Faser (1) zusammenzuarbeiten, entlang der erfassenden optischen Faser (1), um das optische Signal bereitzustellen;
Detektieren der Lichtmenge in dem optischen Signal mit dem Lichtdetektor (7), der mit einem der Enden (9, 10) der optischen Faser (1) gekoppelt ist, während einer vorbestimmten Messzeitdauer;
Benutzen der detektierten Lichtmenge in dem optischen Signal, um die Proteaseaktivität in dem Wundfluid zu bestimmen.

12. Verfahren nach Anspruch 11, wobei das von der Lichtquelle (6) übertragene optische Fasersignal Wellenlängen ungefähr im Bereich des maximalen Absorptionspeaks der lichtabsorbierenden Farbe (5) in der Beschichtung (4).

13. Verfahren nach Anspruch 11 oder 12, wobei das von der Lichtquelle (6) übertragene optische Signal Wellenlängen, die der minimalen Absorption der Licht absorbierenden Farbe (5) in der Beschichtung (4) entspricht, aufweist, um so eine optisches Referenzsignal für die Detektierung der Proteaseaktivität bereitzustellen.

14. Verfahren zum vorbereiten einer optischen Komponente (100) nach Ansprch 1, aufweisend die Schritte von :
Entfernen Ummantelung eines ummantelten Bereichs (2) einer optischen Faser (21) um den Faserkern freizulegen (3);
Beschichten des Faserkerns (3) mit einer Beschichtung (4), wobei die Beschichtung so ausgebildet ist, dass sie sich bei Kontakt mit Protease zersetzt und dass sie ausgebildet ist, eine vorbestimmte Sensitivität auf Protease zu haben, die so ist, dass die Beschichtung (4) sich mit einer vorbestimmten Rate bei Kontakt mit der Protease zersetzt; und
Bereitstellen der Beschichtung (4) mit einer lichtabsorbierenden Farbe (5), die ausgebildet ist, Licht, das in dem Faserkern (3) übertragen wird, zu absorbieren, so dass die Zersetzung der Beschichtung die Lichtmenge, die in dem Faserkern (3) absorbiert werden kann, abnimmt.

15. Verfahren nach Anspruch 14, weiter aufweisend vor dem Beschichten des Faserkerns (3) den Schritt des Aktivierens der Oberfläche des Faserkerns (3) mit einem Aminosilan.

## Revendications

1. Un composant optique (100) pour utilisation dans un capteur optique pour la surveillance d'activité protéasique dans un liquide d'une plaie, le composant optique (100) comprenant:
une fibre optique de détection (1) comprenant une partie non modifiée (2) et une partie modifiée (3), selon laquelle la partie modifiée (3) comprend un coeur de fibre (3), qui est revêtu d'un revêtement (4), le revêtement (4) étant configuré de sorte qu'il se dégrade au contact de la protéase et étant configuré pour avoir une sensibilité prédéterminée à la protéase telle que le revêtement (4) se dégrade à un degré prédéterminé au contact de la protéase,
**caractérisé en ce que** le revêtement (4) comporte un colorant (5) qui est configuré pour absorber la lumière qui est transmise dans le coeur de fibre (3), selon lequel la dégradation du revêtement (4) diminue la quantité de lumière qui peut être absorbée dans la fibre optique de détection (1).

2. Un composant optique (100) selon la revendication 1, selon lequel le revêtement (4) comprend de la gélatine ou des polymères naturels ou synthétiques.

3. Un composant optique (100) selon la revendication 1 ou 2, selon lequel les fibres avec revêtement (4) sont configurées pour avoir une densité réticulée de sorte que le revêtement (4) a une sensibilité prédéterminée au degré de dégradation par la protéase.

4. Un composant optique (100) selon l'une des revendications 1 à 3, selon lequel le revêtement (4) est configuré pour avoir une épaisseur prédéterminée de sorte que le revêtement (4) a une sensibilité de temps de réponse prédéterminée à la protéase.

5. Un composant optique (100) selon l'une des revendications précédentes, selon lequel le colorant (5) comprend de la chlorophylline (5) et/ou de l'éosine.

6. Un pansement comprenant un composant optique (100) selon l'une des revendications 1 à 5.

7. Un dispositif de capteur optique (20) pour la surveillance in situ et continue de l'activité protéasique dans un liquide d'une plaie, selon lequel le dispositif de capteur optique (20) comprend:
un composant optique selon l'une des revendications 1 à 5;
une source de lumière (6) couplée avec une extrémité (9, 10) de la fibre optique capteur (1) de telle sorte que la lumière générée par la source de lumière (6) peut être guidée au travers de la fibre optique capteur (1) pour générer un signal optique qui comprend une lumière qui peut être absorbée par le colorant (59) de la fibre optique capteur;
un détecteur de lumière (7) couplé avec une des extrémités (9, 10) de la fibre optique (1) de sorte que le détecteur de lumière (7) peut recevoir le signal optique;
un module de traitement de signal (37) configuré pour traiter le signal optique reçu par le détecteur de lumière (7) pour déterminer l'activité protéasique se basant sur la quantité de lumière dans le signal optique reçue par le détecteur de lumière.

8. Un dispositif de capteur optique (20) selon la revendication 7, comprenant d'autre part au moins une autre fibre optique capteur (1).

9. Un dispositif de capteur optique (20) selon la revendication 8, selon lequel le revêtement (4) de la fibre optique capteur (1) et le revêtement (4) de ladite au moins une autre fibre optique capteur (1) sont chacun configurés pour avoir des sensibilités prédéterminées différentes à la protéase, de sorte que le revêtement (4) de la fibre optique capteur (1) se dégrade à un degré différent du grade auquel le revêtement (4) de ladite au moins une autre fibre optique capteur (1) se dégrade au contact de la même concentration en protéase.

10. Un dispositif de capteur optique (20) selon la revendication 8 ou 9, selon lequel le module de traitement de signal (37) est configuré de sorte qu'il peut effectuer le multiplexage pour que le module de traitement puisse traiter, individuellement, le signal optique reçu par le détecteur de lumière (7) de la fibre optique capteur (1) et le signal optique reçu par le détecteur de lumière (7) de ladite au moins une autre fibre optique capteur (1) pour déterminer l'activité protéasique.

11. Une méthode pour détecter l'activité protéasique dans un liquide d'une plaie en utilisant un dispositif de capteur optique (20) selon l'une des revendications 7 à 10, comprenant:
disposer le composant optique (100) de sorte que le revêtement (4) de la fibre optique capteur (1) est en contact avec le liquide de la plaie;
transmettre un signal optique de la source optique (6) qui est couplée avec une extrémité de la fibre optique capteur (1) au travers de la fibre optique capteur (1) pour fournir le signal optique;
détecter la quantité de lumière dans le signal optique avec le détecteur de lumière (7) qui est couplé avec une des extrémités (9, 10) de la fibre optique (1) pendant une période de temps de mesure prédéterminée;
utiliser la quantité de lumière détectée dans le signal optique pour déterminer l'activité protéasique dans le liquide de la plaie.

12. Méthode selon la revendication 11, selon laquelle ledit signal de fibre optique transmis par la source de lumière (6) comprend des longueurs d'ondes à environ le pic d'absorption maximum de la lumière qui absorbe le colorant (5) dans le revêtement (4).

13. Méthode selon la revendication 11 ou 12, selon laquelle le signal optique transmis par la source de lumière (6) comprend des longueurs d'ondes correspondant à l'absorption minimum de la lumière qui absorbe le colorant (5) dans le revêtement (4), pour fournir un signal optique de référence de ladite détection d'activité protéasique.

14. Méthode pour préparer le composant optique (100) selon la revendication 1, comprenant les étapes suivantes:
retirer la gaine d'une portion gainée (2) d'une fibre optique (21) pour fournir le coeur de fibre (3);
revêtir le coeur de fibre (3) d'un revêtement (4), selon lequel le revêtement est configuré de telle sorte qu'il se dégrade au contact de la protéase et étant configuré pour avoir une sensibilité prédéterminée à la protéase telle que le revêtement (4) se dégrade à un degré prédéterminé au contact avec la protéase ; et
fournir le revêtement (4) avec un colorant absorbant la lumière (5) qui est configuré pour absorber la lumière qui est transmise dans le coeur de fibre (3), tel que la dégradation du revêtement diminue la quantité de lumière qui peut être absorbée par le coeur de fibre (3).

15. Méthode selon la revendication 14, comprenant d'autre part l'étape, précédant audit revêtement de coeur de fibre (3) d'activation de la surface du coeur de fibre (3) avec un silane aminé.
